**Europäisches Patentamt**

**European Patent Office**

**Office européen des brevets**

(19)

(11) Veröffentlichungsnummer : **0 372 413 B1**

(12)

# EUROPÄISCHE PATENTSCHRIFT

(45) Veröffentlichungstag der Patentschrift :
**09.11.94 Patentblatt 94/45**

(51) Int. Cl.$^5$ : **G01N 33/531,** G01N 33/545, G01N 33/543

(21) Anmeldenummer : **89122198.8**

(22) Anmeldetag : **01.12.89**

(54) **Mittel für immunchemische Tests, carboxylgruppenhaltige Polymere enthaltend.**

(30) Priorität : **02.12.88 DE 3840605**

(43) Veröffentlichungstag der Anmeldung :
**13.06.90 Patentblatt 90/24**

(45) Bekanntmachung des Hinweises auf die
Patenterteilung :
**09.11.94 Patentblatt 94/45**

(84) Benannte Vertragsstaaten :
**AT BE CH DE ES FR GB IT LI LU NL SE**

(56) Entgegenhaltungen :
**EP-A- 0 163 393**
**GB-A- 2 079 936**

(73) Patentinhaber : **BEHRINGWERKE**
**Aktiengesellschaft**
**Postfach 1140**
**D-35001 Marburg (DE)**

(72) Erfinder : **Noah, Michael, Dr.**
**Pappelweg 3**
**D-3550 Marburg (DE)**
Erfinder : **Schmidtberger, Rudolf, Dr.**
**Salegrund 1**
**D-3550 Marburg (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Mittel zum immunchemischen Nachweis und zur Bestimmung eines Analyten im einem biologischen Material, wobei dieses Mittel ein wasserlösliches carboxylgruppenhaltiges Polymer enthält.

Bekannte immunchemische Testsysteme gebrauchen Zusätze von Proteinen, Polysacchariden und/oder Tensiden, die nicht an der immunchemischen Reaktion beteiligt sind, die jedoch geeignet sind, das Ergebnis einer solchen Reaktion günstig zu beeinflussen.

Für Immunoassays, beispielsweise ELISA, müssen die erforderlichen Inkubationsmedien (Pufferlösungen, Inkubationsmilieus, Konjugatpuffer) so zusammengesetzt sein, daß die unspezifische Bindung von Begleitsubstanzen der Probe und/oder des Konjugats an die Festphase verhindert wird. Deshalb werden in den Inkubationsmedien die bekannten Zusätze, wie Proteine, z. B. Albumin, IgG, Kasein, hydrolysierte Gelatine und deren Derivate und Mischungen von Proteinen oder auch humane oder tierische Seren sowie Tenside verwendet.

In DE 36 38 767 ist ein Inkubationsmedium für festphasenimmunchemische Tests beschrieben, das Lactoferrin, foetales Kälberserum, Polyoxiethylen-20-sorbitanmonolaureat ($^R$Tween 20) und Puffersalze enthält.

In EP-A 215 457 sind ebenfalls Zusätze von Tensiden aus der Gruppe der Poloxamere, beispielsweise $^R$Pluronic F 68 und aus der Gruppe der Poloxamine, beispielsweise $^R$Tetronic 707 und 1107 beschrieben, wobei die drei genannten Verbindungen als vorteilhaft gegenüber $^R$Tween 20 herausgestellt sind.

Mit diesen Zusätzen versehene Inkubationsmedien können aber falsche Meßwerte nicht verhindern, welche z. B. in Sandwich-Einschritt-Testen auftreten, die zur Erzielung der optimalen Empfindlichkeit hohe Probenmengen bei hohen Konjugatkonzentrationen, aber geringen Konjugatvolumina benutzen.

Es bestand daher die Aufgabe, ein Mittel zu finden, mit dem durch die Inkubation mit der Probe falsche Meßwerte verhindert werden.

Es wurde überraschend gefunden, daß in Wasser lösliche carboxylgruppenhaltige Polymere noch besser geeignet sind als Zusätze des Standes der Technik, falsche Meßwerte zu verhindern und eine immmunchemische Reaktion günstig zu beeinflussen, was eine höhere Empfindlichkeit des Nachweises und der Bestimmung eines in einem auch als Probe zu bezeichnenden biologischen Material enthaltenen Analyten bewirkt.

Diese Polymere als Bestandteil eines solchen Mittels können bereits bei der Vorbereitung der Probe für den Test vorhanden sein.

Gegenstand der Erfindung ist daher ein Mittel zum Nachweis oder zur Bestimmung eines Analyten in einem biologischen Material enthaltend immunchemische Reaktanten, von denen mindestens einer mit dem Analyten reagiert dadurch gekennzeichnet, daß es weiterhin ein in Wasser lösliches carboxylgruppenhaltiges Polymer enthält.

Gegenstand der Erfindung ist weiterhin die Verwendung eines solchen Mittels in immunchemischen Tests.

In der GB-A 2079936 werden Mikrokapseln als Reagenzträger beschrieben, die unter anderem aus carboxylgruppenhaltigen Polymeren bestehen können. Diese Kapseln sind in wässrigen Lösungen stabil.

Geeignete wasserlösliche carboxylgruppenhaltige Polymere können aus wasserunlöslichen synthetischen Polymeren, die Ester- oder Anhydridgruppen tragen, durch alkalische Hydrolyse hergestellt werden. Solche wasserunlöslichen Polymere sind bekannt. Beispiele dafür sind Polymere, bei deren Herstellung als Monomere Ester von Acrylsäure und Alkylacrylsäure oder Maleinsäureanhydrid alleine oder in Mischung mit Monomeren, die keine Carboxylgruppen enthalten (beispielsweise Methylvinyläther, Styrol, Ethylen, Propylen, Octadecen), verwandt werden Diese als Homopolymere oder als Heteropolymere zu bezeichnenden Polymere werden im allgemeinen durch radikalische Polymerisation, d. h. durch Zusatz von Radikalinitiatoren, beispielsweise durch Cumarylhydroperoxid oder durch Dibenzoylperoxid erhalten, wobei die Kettenlänge des Polymers durch die zugegebene Menge des Peroxids, durch die Temperatur oder den Zusatz eines Telogens, beispielsweise von Tetrachlorkohlenstoff oder einem Radikalfänger, beispielsweise von N-Acetylcysteinsäure oder einem anderen Thiol gesteuert wird.

Als Polymere werden im allgemeinen Reaktionsprodukte bezeichnet, deren Molekulargewicht größer als 2000 ist, als Oligomere oder Telomere solche Produkte mit einem Molekulargewicht kleiner als 2000.

Die Herstellung von wasserlöslichen carboxylgrvppenhaltigen synthetischen Heteropolymeren gelingt nur dann, wenn das Verhältnis von carboxylgruppentragendem Monomer und den Monomeren, die keine Carborylgruppen enthalten, so gewählt wird, daß das erhaltene Polymer, Oligomer oder Telomer nach alkalischer Hydrolyse wasserlöslich ist. Dieses Verhältnis ist für jedes Heteropolymer empirisch zu bestimmen. Z. B. ist ein Heteropolymer, hergestellt aus den Monomeren Styrol und Maleinsäureanhydrid bei einem molaren Verhältnis 2 bis 2.5 zu 1 nach alkalischer Hydrolyse noch wasserlöslich, bei einem Verhältnis über 3 zu 1 bleibt

2

es nach alkalischer Hydrolyse wasserunlöslich. Geeignet sind auch wasserlösliche carboxylgruppenhaltige Polypeptide enthaltend Asparaginsäure, Glutaminsäure ggf. auch neutrale Aminosäuren, die im Handel erhältlich sind oder durch bekannte Methoden der Peptidsynthese herstellbar sind.

Außerdem sind geeignet die aus Naturprodukten erhältlichen Zuckersäuren enthaltenden Polysaccharide, beispielsweise Pektinsäure und Galacturonsäure.

Folgende Polymere werden bevorzugt:

Polyacrylsäure, Polymaleinsäure, Polymerisate oder Telomerisate von Maleinsäureanhydrid und Methylvinyläther oder Ethylen oder Propylen oder Octadecen, deren Anhydridringe durch Hydrolyse geöffnet werden, Polyasparaginsäure, Polyglutaminsäure und Polygalacturonsäure.

Besonders bevorzugt werden Telomerisate von Maleinsäureanhydrid und Methylvinyläther oder Propylen, die durch Behandlung mit Natronlauge in die wasserlöslichen carboxylhaltigen Polymere überführt werden.

Es sind eine Vielzahl von Mitteln enthaltend immunchemische Reaktanten und/oder bioaffine Bindungspartner bekannt.

Die Mittel werden im allgemeinen danach bezeichnet, für welche immunchemischen Verfahren sie verwandt werden.

Mittel im Sinne der Erfindung sind solche, mit denen Präzipitate als Dispersion oder in einem Gel oder Agglutinate von Partikeln erzeugt oder deren Ausbleiben bewirkt werden, oder solche, bei denen durch die immunchemische Reaktion ein Farbsignal oder eine Strahlung erzeugt oder verhindert wird.

Aus der Gruppe der letztgenannten Mittel werden solche bevorzugt, mit denen festphasenimmunchemische Tests durchgeführt werden, die bei Erzeugung einer Färbung oder einer Strahlung aus einem Enzymsubstrat als ELISA (enzyme linked immunosorbent assay) oder als Scintillations-Assay, bei Erzeugung einer Strahlung durch ein radioaktiv markiertes Isotop als Festphasen-Radioimmunoassay oder bei Erzeugung einer Fluoreszenz durch ein Fluorogen als Festphasen-Fluoreszenzassay bezeichnet werden.

Diagnostische Mittel enthalten Antigen, Antikörper oder zugleich beide als Reaktanten, sowie andere für die Reaktanten oder auch den Analyten bioaffine Bindungspartner, beispielsweise Lektine, Komplement, Protein A oder G, sowie derivatisiertes Biotin und Avidin, wobei mindestens einer der Reaktanten mit einer Markierung versehen sein kann und gegebenenfalls Reagenzien zum Nachweis der Markierung. Außerdem kann einer der Reaktanten als Festphase vorliegen.

Eine Festphase im Sinne der Erfindung ist ein wasserunlöslicher Träger, an dem ein oder mehrere Reaktanten gebunden sind.

Träger sind beispielsweise Latexpartikel, körniges, quellbares oder nicht quellbares Material, Kugeln, Innenflächen von Röhrchen, Mikrotestplatten als besondere Ausführungsform einer Anordnung von Röhrchen und auch als saugfähige Matrix zu bezeichnende poröse Materialien.

Ein Bestandteil des erfinddunggemäßen Mittels kann eine Vorrichtung zur Aufnahme einer Probe, beispielsweise ein Gefäß zur Probenaufnahme oder die Aufnahmezone, beispielsweise eine saugfähige Matrix, für die Probe auf einem sogenannten "trockenchemischen" Testsystem sein. Der Bestandteil kann auch eine wässrige Lösung sein, in der auch Puffersalze und ggf. stabilisierende Zusätze wie Proteine oder Polysaccharide als ebenfalls für den Analyten stabilisierende Substanzen enthalten sind, wobei das Polymer in einer Konzentration von 0.01 bis 50 g/l enthalten ist, bevorzugt von 0.1 bis 20 g/l, besonders bevorzugt von 0.2 bis 2 g/l.

Das Polymer kann auch in einer Vorrichtung als Bestandteil des diagnostischen Mittels enthalten sein, in der die immunchemische Reaktion stattfindet.

Die auch als Probe bezeichneten biologischen Materialien, die den Analyten enthalten, sind beispielsweise Gewebe von Biopsien oder Autopsien, Blut, Blutzellen, Serum oder Plasma, Sekrete, Liquor, aus entzündetem oder nicht entzündetem Gewebe, die Zerfallsprodukte von Gewebe und Stoffwechselausscheidungen.

Gegenstand der Erfindung ist weiterhin ein Verfahren zur immunchemischen Bestimmung eines in einem biologischen Material enthaltenen Analyten, dadurch gekennzeichnet, daß man den Analyten mit dem Polymer zusammenbringt ggf. mit einer wässrigen Lösung inkubiert und mit dem derart erhaltenen Gemisch eine immunchemische Bestimmung durchführt.

Die biologischen Materialien werden mit dem Polymer zusammengebracht und können derart überlängere Zeit gelagert werden, ohne daß sich der darin enthaltene Analyt in seinen immunchemischen Eigenschaften verändert.

Bevorzugt werden immunchemische Verfahren, bei denen einer der Reaktanten in fester Phase vorliegt, wobei das zuvor beschriebene, behandelte Material mit der festen Phase zusammengebracht wird, gegebenenfalls zusammen mit weiteren immunchemischen Reaktanten außer denen der festen Phase und Reagenzien zum Nachweis des Analyten, wobei anschließend die feste Phase von der flüssigen Phase getrennt und der Analyt entweder an der festen oder in der flüssigen Phase bestimmt wird.

Beispiele:

1.Herstellung von wasserlöslichen carboxlhaltigen Polymeren

1.1 Carboxylat von Maleinsäureanhydrid-Propylen-Telomerisat (MPT-Carboxylat)

5 g MPT 155, ein Handelsprodukt der Stickstoffwerke Linz, wurden in 500 ml entionisertem Wasser suspendiert. Dann wurden innerhalb einer Stunde 13 ml 5 normale Natronlauge unter Rühren zugetropft. Es wurde weitere 3 Stunden gerührt, wobei ein pH-Wert von 8 durch weitere Zugabe von 1 normaler Natronlauge gehalten wurde. Die derart erhaltene Lösung wurde noch weitere 15 Stunden bei 20 - 25 °C stehen gelassen, bevor sie weiter verwendet wurde.

1.2 Carboxylat von Maleinsäureanhydrid-Methylvinyläther-Telomerisat (MMVT-Caboxylat)

5 g eines unter der Bezeichnung Gantrez AN179 von der Firma Serva, Heidelberg, erhältliches Maleinsäureanhydrid-Methylvinyläther-Telomerisat wurde behandelt wie bei 1.1 beschrieben.

2. Herstellung von Testbestecken für die Bestimmung von Hepatitis B-surface-Antigen (HBs) nach dem Festphasen-2-Seiten-immunchemischen Verfahren (Sandwich-ELISA)

2.1 Mikrotestplatten, beschichtet mit Antikörpern gegen HBs

Mikrotestplatten, d. h. Immunoplatten II 96 F mit rundem Boden (Firma Nunc, Roskilde, Dänemark, Artikel Nr. 262162) wurden mit monoklonalen Anti-HBs und Immunglobulin G (IgG) von der Maus beschichtet. Dazu wurde das IgG auf 2 mg/l in 100 mmol/l Natriumbicarbonat pH 9.6 verdünnt. In jede Vertiefung (Well) der Mikrotestplatten wurden 100 µl der Verdünnung gegeben. Die derart gefüllten Testplatten wurden 18 Stunden bei 20°C belassen, dann wurden 3-4 mal mit 200 µl einer Lösung von 1 g/l $^R$Tween 20 in phosphatgepufferter physiologischer Kochsalzlösung, pH 7.4 durch Füllen und Absaugen gewaschen und die Testplatten anschließend über Kieselgel bei 20 °C getrocknet.

2.2 Anti-HBs IgG-Peroxidase Konjugat

Monoklonales Maus-IgG, gerichtet gegen HBs, wurde mit N-gamma-Maleinmidobutryloxisuccinimid (GMBS) umgesetzt wie von Tanamori et al., 1983 in J. Immunol. Meth. 62, 123-131 beschrieben. 2-Iminothiolanhydrochlorid (Fa. Sigma, Kt. Nr. I 6256) wurde mit Meerrettich-Peroxidase (POD), bezogen von der Fa. Boehringer Mannheim, Kat. Nr. 413470, umgesetzt wie von King et al. 1978 in Biochem. 17, 1499-1506 beschrieben. Aus dem GMBS-IgG Konjugat und dem Iminothiolan-POD Konjugat wurde ein IgG-POD Konjugat hergestellt wie von Tanamori beschrieben. Die erhaltene Lösung des IgG-POD Konjugats hatte einen Proteingehalt von 1.2 mg/ml. Das Verhältnis von POD zu IgG wurde mit 2.5 bestimmt. Die Lösung wurde anschließend auf 24 und 6 µg/ml IgG-POD mit einer Lösung verdünnt, die 200 ml/l durch Erhitzen defibriniertes und delipidiertes Rinderserum, 1 g/l $^R$Polygeline, 50 mmol/l Tris, 150 mmol/l Trinatriumcitrat, 0.5 mol/l NaCl, eingestellt mit HCl auf pH 7.4, und wurde als 24 µl/ml, bzw. 6 µg/ml Anti-HBs-POD bezeichnet.

2.3 TMB-Substratzubereitung

Zum Nachweis von Anti-HBS-POD wurde ein Substratsystem oder eine Substratzubereitung verwandt, enthaltend Wasserstoffperoxid und Tetramethylbenzidin (TMB), welche aus zwei Stammlösungen hergestellt wurde.
Stammlösung 1: TMB-Dihydrochlorid wurde unter Rühren in einer Konzentration von 5 g/l, d. h. von 16 mmol/l in bidestilliertem Wasser gelöst und mit normaler Salzsäure auf pH 1.5 eingestellt. Zu dieser Lösung wurde Penicilin G unter Rühren in einer Endkonzentration von 200 mg/l, d. h. von 0.56 mmol/l zugesetzt.
Stammlösung 2: zu 900 ml bidestilliertem Wasser wurde 1.4 ml Eisessig, 1.5 ml 1 normale NaOH und 250 mg, d. h. 3 mmol $H_2O_2$ als Harnstoff-Wasserstoff-peroxid-Addukt zugesetzt. Nach vollständigem Lösen wurde mit bidestilliertem Wasser auf 1 l aufgefüllt.
TMB-Substratzubereitung: Ein Volumenteil der Stammlösung 1 und 10 Volumenteile der Stammlösung 2 wurden miteinander vermischt.

3. Vergleichende Bestimmungen und Ergebnisse

3.1 Bestimmung von HBs mit dem Einschritt-Sandwich-ELISA

In die Bestimmung wurden einige Seren einbezogen, die nach einem Verfahren des Standes der Technik, nämlich dem Zweischritt-Sandwich-ELISA als HBs-negativ bestimmt worden waren, die aber in dem Einschritt-Sandwich-ELISA ohne die Verwendung eines carboxylgruppenhaltigen Polymeren nicht eindeutig zu bewerten waren, weil sie Extinktionen größer als 0.04 erzeugten. Da alle als HBs-negativ eingestuften Proben eines Referenzpanels in dem Zweischritt-Sandwich-ELISA Extinktionen kleiner als 0.040 erzeugten, wurde dieser ELISA als zuverlässig hinsichtlich Vermeidung falsch positiver Ergebnisse gewertet. Bei der Bestimmung wurden außerdem eine negative und eine positive Kontrolle mitgeführt. Für jede Kontrolle und für jede Probe wurden 2 Vertiefungen einer Mikrotestplatte, die, wie unter 2.1 beschrieben, mit Antikörpern gegen HBs beschichtet worden waren, benutzt. In Gruppen von 2 Vertiefungen wurden 25 µl einer Lösung von 24 µg/ml Anti-HBsPOD sowie 25 µl dieser Lösung mit dem Zusatz von 1 g/l MMVT-Carboxylat. Pro Vertiefung jeder Gruppe wurden entweder 100 µl negative oder 100 µl positive Kontrolle oder 100 µl Probe eingefüllt.

Die derart behandelte Mikrotestplatte wurde 1 h bei 37 °C stehen gelassen. Dann wurde der Inhalt der Vertiefungen durch Absaugen entfernt und die Vertiefungen 4 mal mit jeweils 200 µl einer Lösung von 1 g/l RTween 20 in phosphatgepufferter physiologischer Kochsalzlösung, pH 7.4, durch Füllen und Absaugen gewaschen. Es wurden in jede Vertiefung 100 µl TMB-Substratzubereitung gegeben, 30 min bei 20 - 22 °C stehen gelassen und dann 100 µl 1-normale Schwefelsäure zugegeben. Die Extinktionen der Lösungen in den Vertiefungen wurden gegen einen Leerwert von 200 µl phosphatgepufferter Kochsalzlösung in einer weiteren Vertiefung bei 450 mm gemessen.

3.2 Bestimmung von HBs mit dem Zweischritt-Sandwich-ELISA, einem Verfahren des Standes der Technik

In Vertiefungen einer Miktrotestplatte, beschichtet mit Antikörpern gegen HBs gemäß Beispiel 2.1 wurden 100 µl negative Kontrolle, 100 µl positive Kontrolle und 100 µl der Proben, die auch in dem Einschritt-Sandwich getestet wurden, eingefüllt. Die Platte wurde 1 h bei 37 °C stehen gelassen. Danach wurde der Inhalt der Vertiefungen 4 mal gewaschen, wie in Beispiel 3.1 beschrieben. Anschließend wurden 100 µl einer Lösung von 6 µg/ml Anti-HBs-POD gegeben und die Platte wurde wieder 1 h bei 37 °C stehen gelassen. Der Inhalt der Vertiefungen wurde entfernt und die Vertiefungen gewaschen, wie zuvor beschrieben. Die Zugabe von TMB-Substratzubereitung und von Schwefelsäure sowie die Messung der Extinktion der Lösungen in den Vertiefungen erfolgte in der gleichen Weise wie in Beispiel 3.1 beschrieben.

3.3 Messergebnisse der Bestimmungen von HBs gemäß den Beispielen 3.1 und 3.2

In der Tabelle sind die Extinktionen, erhalten von der negativen, der positiven Kontrolle und von 10 Serumproben nach dem Einschritt- und dem Zweischritt-Sandwich-ELISA dargestellt. Außerdem sind Werte für die als "cut-off" bezeichnete Obergrenze der Extinktion von als HBs-negativ einzustufenden Proben angegeben. Der "cut-off" wurde festgelegt als die Extinktion der negativen Kontrolle plus einer Extinktion von 0.025.

Aus der Tabelle wird ersichtlich, daß der Einschritt-Sandwich-Assay für die positive Kontrolle höhere Extinktionen liefert als der Zweischritt-Sandwich-ELISA, daß aber die bei dem im Zweischritt-Assay als negativ ermittelten Proben dem Einschritt-Assay dann als positiv erscheinen, wenn die Lösung, die das HBs-POD Konjugat enthält, keinen Zusatz von MMVT-Carboxylat enthält.

Der beschriebene Einschritt-Sandwich-ELISA enthaltend MMVT-Carboxylat ist bei höher Empfindlichkeit zuverlässig im Hinblick auf die Entscheidung, ob eine Probe als HBs negativ einzustufen ist, weil die gemessenen Extinktionen alle unter 0.040, dem "cutt-off" liegen und nicht etwa bei 0.040.

In weiteren Versuchen wurde gezeigt, daß Einschritt-Sandwich-ELISAs zum Nachweis von HBs enthaltend MPT-Carboxylat, Polyacrylsäure, Polyasparaginsäure und/oder Polyglacturonsäure gleichwertige Ergebnisse zeigen.

Tabelle

Sandwich - ELISA

| | Einschritt mit MMVT-Carboxylat | Einschritt | Zweischritt |
|---|---|---|---|
| | Extinktionen bei 450 nm | | |
| negative Kontrolle | 0.015 | 0.014 | 0.019 |
| cutt-off | 0.040 | 0.039 | 0.044 |
| positive Kontrolle | 1.692 | 1.514 | 0.918 |
| HBs neg. Seren | | | |
| 1 | 0.018 | 0.281 | 0.038 |
| 2 | 0.011 | 0.076 | 0.018 |
| 3 | 0.008 | 0.072 | 0.028 |
| 4 | 0.005 | 0.024 | 0.023 |
| 5 | 0.007 | 0.128 | 0.028 |
| 6 | 0.020 | 0.017 | 0.025 |
| 7 | 0.030 | 0.011 | 0.017 |
| 8 | 0.036 | 0.049 | 0.015 |
| 9 | 0.010 | 0.056 | 0.029 |
| 10 | 0.036 | 0.015 | 0.026 |

**Patentansprüche**

**Patentansprüche für folgende Vertragsstaaten : AT, BE, CH, DE, FR, GB, IT, LI, LU, NL**

1.   Mittel zum Nachweis oder zur Bestimmung eines Analyten in einem biologischen Material enthaltend immunchemische Reaktanten, von denen mindestens einer mit dem Analyten reagiert, dadurch gekennzeichnet, daß es weiterhin ein carboxylgruppenhaltiges wasserlösliches Polymer enthält.

2. Mittel nach Anspruch 1, dadurch gekennzeichnet, daß das carboxylgruppenhaltige Polymer Polyacrylsäure ist.

3. Mittel nach Anspruch 1, dadurch gekennzeichnet, daß das carboxylgruppenhaltige Polymer durch alkalische Hydrolyse von Maleinsäureanhydrid-Propylen-Telomerisat oder Polymerisat erhalten wird.

4. Mittel nach Anspruch 1, dadurch gekennzeichnet, daß das carboxylgruppenhaltige Polymer durch alkalische Hydrolyse von Maleinsäureanhydrid-Methylvinyläther-Telomerisat oder Polymerisat erhalten wird.

5. Mittel nach Anspruch 1, dadurch gekennzeichnet, daß sich das carboxylgruppenhaltige Polymer in einer Lösung befindet.

6. Mittel nach Anspruch 1, dadurch gekennzeichnet, daß sich das carboxylgruppenhaltige Polymer in einem trockenchemischen Testsystem befindet.

7. Ein Verfahren zur immunchemischen Bestimmung eines in einem biologischen Material enthaltenen Analyten, dadurch gekennzeichnet, daß man den Analyten mit dem carboxylgruppenhaltigen Polymer nach Anspruch 1 zusammenbringt, gegebenenfalls mit einer wässrigen Lösung inkubiert und mit dem derart erhaltenen Gemisch eine immunchemische Bestimmung durchführt.

8. Verfahren zur immunchemischen Bestimmung eines in einem biologischen Material enthaltenen Analyten, dadurch gekennzeichnet, daß man ein carboxylgruppenhaltiges Polymer nach Anspruch 1 als Lösung, die gegebenenfalls immunchemische Reaktanten enthält, mit dem Analyten und mit einer als Festphase vorliegenden weiteren immunchemischen Reaktanten zusammenbringt, anschließend die feste Phase von der flüssigen Phase trennt und den Analyten entweder an der festen Phase oder in der flüssigen Phase bestimmt.

9. Verfahren zur immunchemischen Bestimmung eines in einem biologischen Material enthaltenen Analyten, dadurch gekennzeichnet, daß man in einem Mittel nach Anspruch 1 das in trockener Form vorliegende carboxylgruppenhaltige Polymer mit einer wässrigen Lösung, die gegebenenfalls den Analyten und/oder immunchemische Reaktanten enthält, zusammenbringt, die erhaltene Lösung gleichzeitig oder darauffolgend mit einem als Festphase vorliegenden weiteren immunchemischen Reaktanten kontakiert und den Analyten entweder an der festen oder in der flüssigen Phase bestimmt.

10. Verwendung eines Mittels nach Anspruch 1 in immunchemischen Tests.

**Patentansprüche für folgenden Vertragsstaat : ES**

1. Verfahren zur Herstellung eines Mittels zum Nachweis oder zur Bestimmung eines Analyten in einem biologischen Material, wobei
mindestens ein immunchemischer Reaktand, der mit dem Analyten reagiert, in einer wässrigen Lösung mit einem carboxylgruppenhaltigen wasserlöslichen Polymer in Kontakt gebracht wird.

2. Verfahren nach Anspruch 1 dadurch gekennzeichnet, daß das carboxylgruppenhaltige Polymer Polyacrylsäure ist.

3. Verfahren nach Anspruch 1, dadurch gekennzeichnet, daß das carboxylgruppenhaltige Polymer durch alkalische Hydrolyse von Maleinsäureanhydrid-Propylen-Telomerisat oder Polymerisat erhalten wird.

4. Verfahren nach Anspruch 1, dadurch gekennzeichnet, daß das carboxylgruppenhaltige Polymer durch alkalische Hydrolyse von Maleinsäureanhydrid-Methylvinyläther-Telomerisat oder Polymerisat erhalten wird.

5. Verfahren nach Anspruch 1, dadurch gekennzeichnet, daß sich das carboxylgruppenhaltige Polymer in einer Lösung befindet.

6. Verfahren nach Anspruch 1, dadurch gekennzeichnet, daß sich das carboxylgruppenhaltige Polymer in einem trockenchemischen Testsystem befindet.

7. Verfahren zur immunchemischen Bestimmung eines in einem biologischen Material enthaltenen Analyten, dadurch gekennzeichnet, daß man den Analyten mit dem carboxylgruppenhaltigen Polymer nach Anspruch 1 zusammenbringt, gegebenenfalls mit einer wässrigen Lösung inkubiert und mit dem derart erhaltenen Gemisch eine immunchemische Bestimmung durchführt.

8. Verfahren zur immunchemischen Bestimmung eines in einem biologischen Material enthaltenen Analyten, dadurch gekennzeichnet, daß man ein carboxylgruppenhaltiges Polymer nach Anspruch 1 als Lösung, die gegebenenfalls immunchemische Reaktanten enthält, mit dem Analyten und mit einer als Festphase vorliegenden weiteren immunchemischen Reaktanten zusammenbringt, anschließend die feste Phase von der flüssigen Phase trennt und den Analyten entweder an der festen Phase oder in der flüssigen Phase bestimmt.

9. Verfahren zur immunchemischen Bestimmung eines in einem biologischen Material enthaltenen Analyten, dadurch gekennzeichnet, daß man in einem Mittel nach Anspruch 1 das in trockener Form vorliegende carboxylgruppenhaltige Polymer mit einer wässrigen Lösung, die gegebenenfalls den Analyten und/oder immunchemische Reaktanten enthält, zusammenbringt, die erhaltene Lösung gleichzeitig oder darauffolgend mit einem als Festphase vorliegenden weiteren immunchemischen Reaktanten kontakiert und den Analyten entweder an der festen oder in der flüssigen Phase bestimmt.

## Claims

**Claims for the following Contracting States : AT, BE, CH, DE, FR, GB, IT, LI, LU, NL**

1. An agent for the detection or determination of an analysis substance in a biological material containing immunochemical reactants, at least one of which reacts with the analysis substance, which agent moreover contains a water-soluble polymer containing carboxyl groups.

2. An agent as claimed in claim 1, wherein the polymer containing carboxyl groups is polyacrylic acid.

3. An agent as claimed in claim 1, wherein the polymer containing carboxyl groups is obtained by alkaline hydrolysis of maleic anhydride-propylene telomer or polymer.

4. An agent as claimed in claim 1, wherein the polymer containing carboxyl groups is obtained by alkaline hydrolysis of maleic anhydride-methyl vinyl ether telomer or polymer.

5. An agent as claimed in claim 1, wherein the polymer containing carboxyl groups is in solution.

6. An agent as claimed in claim 1, wherein the polymer containing carboxyl groups is in a dry chemical test system.

7. A process for the immunochemical determination of an analysis substance contained in a biological material, which comprises bringing the analysis substance together with the polymer containing carboxyl groups, as claimed in claim 1, if appropriate incubating the mixture in an aqueous solution and performing an immunochemical determination with the mixture obtained in this way.

8. A process for the immunochemical determination of an analysis substance contained in a biological material, which comprises bringing together a polymer containing carboxyl groups as claimed in claim 1, as a solution which contains, if appropriate, immunochemical reactants, together with the analysis substance and with another immunochemical reactant present as a solid phase, subsequently separating the solid phase from the liquid phase and determining the analysis substance either on the solid phase or in the liquid phase.

9. A process for the immunochemical determination of an analysis substance contained in a biological material, which comprises bringing the polymer containing carboxyl groups, present in dry form, together with an aqueous solution, which if appropriate contains the analysis substance and/or immunochemical reactants, in an agent as claimed in claim 1, at the same time or subsequently bringing the resulting solution into contact with another immunochemical reactant present as a solid phase, and determining the analysis substance either on the solid or in the liquid phase.

10. The use of an agent as claimed in claim 1 in immunochemical tests.

**Claims for the following Contracting State : ES**

1. A process for the preparation of an agent for the detection or determination of an analysis substance in a biological material, in which at least one immunochemical reactant which reacts with the analysis substance, is brought into contact with a water-soluble polymer containing carboxyl groups, in an aqueous solution.

2. The process as claimed in claim 1, wherein the polymer containing carboxyl groups is polyacrylic acid.

3. The process as claimed in claim 1, wherein the polymer containing carboxyl groups is obtained by alkaline hydrolysis of maleic anhydride-propylene telomer or polymer.

4. The process as claimed in claim 1, wherein the polymer containing carboxyl groups is obtained by alkaline hydrolysis of maleic anhydride-methyl vinyl ether telomer or polymer.

5. The process as claimed in claim 1, wherein the polymer containing carboxyl groups is in solution.

6. The process as claimed in claim 1, wherein the polymer containing carboxyl groups is in a dry chemical test system.

7. A process for the immunochemical determination of an analysis substance contained in a biological material, which comprises bringing the analysis substance together with the polymer containing carboxyl groups, as claimed in claim 1, if appropriate incubating the mixture in an aqueous solution and performing an immunochemical determination with the mixture obtained in this way.

8. A process for the immunochemical determination of an analysis substance contained in a biological material, which comprises bringing together a polymer containing carboxyl groups as claimed in claim 1, as a solution which contains, if appropriate, immunochemical reactants, together with the analysis substance and with another immunochemical reactant present as a solid phase, subsequently separating the solid phase from the liquid phase and determining the analysis substance either on the solid phase or in the liquid phase.

9. A process for the immunochemical determination of an analysis substance contained in a biological material, which comprises bringing the polymer containing carboxyl groups, present in dry form, together with an aqueous solution, which if appropriate contains the analysis substance and/or immunochemical reactants, in an agent as claimed in claim 1, at the same time or subsequently bringing the resulting solution into contact with another immunochemical reactant present as a solid phase, and determining the analysis substance either on the solid or in the liquid phase.

**Revendications**

**Revendications pour les Etats contractants suivants : AT, BE, CH, DE, FR, GB, IT, LI, LU, NL**

1. Milieu pour l'identification ou le dosage d'un analyte dans une substance biologique contenant des réactifs immunochimiques, dont au moins l'un d'entre eux réagit avec l'analyte, ledit milieu étant caractérisé en ce qu'il renferme en plus un polymère carboxylique hydrosoluble.

2. Milieu selon la revendication 1, caractérisé en ce que le polymère carboxylique est le poly(acide acrylique).

3. Milieu selon la revendication 1, caractérisé en ce que le polymère carboxylique est obtenu par hydrolyse alcaline d'un télomère ou polymère anhydride maléique/propylène.

4. Milieu selon la revendication 1, caractérisé en ce que le polymère carboxylique est obtenu par hydrolyse alcaline d'un télomère ou polymère anhydride maléique/oxyde de méthyle et de vinyle.

5. Milieu selon la revendication 1, caractérisé en ce que le polymère carboxylique se trouve en solution.

6. Milieu selon la revendication 1, caractérisé en ce que le polymère carboxylique se trouve dans un système d'essai chimique sec.

7. Procédé de dosage immunologique d'un analyte contenu dans une substance biologique, caractérisé en ce qu'on met l'analyte en contact avec le polymère carboxylique selon la revendication 1, on le fait éventuellement incuber avec une solution aqueuse et on effectue un dosage immunologique avec le mélange ainsi obtenu.

8. Procédé de dosage immunologique d'un analyte contenu dans une substance biologique, caractérisé en ce qu'on met un polymère carboxylique selon la revendication 1, comme solution contenant éventuellement des réactifs immunochimiques, en contact avec l'analyte et avec un autre réactif immunochimique présent sous forme de phase solide puis on sépare la phase solide d'avec la phase liquide et on dose l'analyte soit sur la phase solide, soit dans la phase liquide.

9. Procédé de dosage immunologique d'un analyte contenu dans une substance biologique, caractérisé en ce que, dans un milieu selon la revendication 1, on met le polymère carboxylique à l'état sec en contact avec une solution aqueuse contenant éventuellement l'analyte et/ou des réactifs immunochimiques, on met, simultanément ou successivement, en contact la solution obtenue avec un autre réactif immunochimique présent sous forme solide et on dose l'analyte soit sur la phase solide, soit dans la phase liquide.

10. Utilisation d'un milieu selon la revendication 1 dans des dosages immunologiques.

**Revendications pour l'Etat contractant suivant : ES**

1. Procédé de préparation d'un milieu pour l'identification ou le dosage d'un analyte dans une substance biologique, dans lequel au moins un réactif immunochimique, qui réagit avec l'analyte, est mis en contact en solution aqueuse avec un polymère carboxylique hydrosoluble.

2. Procédé selon la revendication 1, caractérisé en ce que le polymère carboxylique est le poly(acide acrylique).

3. Procédé selon la revendication 1, caractérisé en ce que le polymère carboxylique est obtenu par hydrolyse alcaline d'un télomère ou polymère anhydride maléique/propylène.

4. Procédé selon la revendication 1, caractérisé en ce que le polymère carboxylique est obtenu par hydrolyse alcaline d'un télomère ou polymère anhydride maléique/oxyde de méthyle et de vinyle.

5. Procédé selon la revendication 1, caractérisé en ce que le polymère carboxylique se trouve en solution.

6. Procédé selon la revendication 1, caractérisé en ce que le polymère carboxylique se trouve dans un système d'essai chimique sec.

7. Procédé de dosage immunologique d'un analyte contenu dans une substance biologique, caractérisé en ce qu'on met l'analyte en contact avec le polymère carboxylique selon la revendication 1, on le fait éventuellement incuber avec une solution aqueuse et on effectue un dosage immunologique avec le mélange ainsi obtenu.

8. Procédé de dosage immunologique d'un analyte contenu dans une substance biologique, caractérisé en ce qu'on met un polymère carboxylique selon la revendication 1, comme solution contenant éventuellement des réactifs immunochimiques, en contact avec l'analyte et avec un autre réactif immunochimique présent sous forme de phase solide puis on sépare la phase solide d'avec la phase liquide et on dose l'analyte soit sur la phase solide, soit dans la phase liquide.

9. Procédé de dosage immunologique d'un analyte contenu dans une substance biologique, caractérisé en ce que, dans un milieu selon la revendication 1, on met le polymère carboxylique à l'état sec en contact avec une solution aqueuse contenant éventuellement l'analyte et/ou des réactifs immunochimiques, on met, simultanément ou successivement, en contact la solution obtenue avec un autre réactif immunochimique présent sous forme solide et on dose l'analyte soit sur la phase solide, soit dans la phase liquide.